# EUROPÄISCHE PATENTANMELDUNG

(11) **EP 2 364 743 A1**
(43) Veröffentlichungstag der Anmeldung: **14.09.2011**
(21) Anmeldenummer: 11000933.9
(22) Anmeldetag: 05.02.2011
(51) Int. Cl.: A61M 5/30, A61M 5/178, A61J 1/20

(54) **Verfahren und Vorrichtung zum Befüllen eines Einmalinjektors**

(30) Priorität: 08.03.2010 DE 102010010699
(71) Anmelder: LTS LOHMANN Therapie-Systeme AG, 56626 Andernach (DE)
(72) Erfinder: Matusch, Rudolf, 35041 Marburg (DE)
(74) Vertreter: Schmidt, Werner

(57) **Zusammenfassung**

Die Erfindung betrifft ein Verfahren und eine Vorrichtung zum sterilen Befüllen eines Einmalinjektors mit einer definierten Dosis einer Injektionslösung, wobei der Einmalinjektor eine Zylinder-Kolben-Einheit mit einem Zylinder und mit einem manuell zurückziehbaren Kolben umfasst. Die definierte Dosis der Injektionslösung ist in einem Behälterinnenraum eines Behälters einer in einem Durchgangsstopfen gehaltenen Nadelspritze bevorratet. Der Zylinderinnenraum ist mit dem Behälterinnenraum durch den Durchgangsstopfen hindurch gas- und flüssigkeitsdicht verbunden. Die Zylinder-Kolben-Einheit wird durch Zurückziehen des Kolbens oder Drücken des Nadelspritzenkolbens befüllt.

Mit der vorliegenden Erfindung werden ein Verfahren und eine Vorrichtung zum Befüllen eines Einmalinjektors entwickelt, bei dem ohne Zwischenwiegen oder Gefahr von Ableseungenauigkeiten der Einmalinjektor mit einer definierten Dosis einer Injektionslösung aus einer Spritze mit eingeschmolzener Kanüle befüllt werden kann.

## Beschreibung

Die Erfindung betrifft ein Verfahren und eine Vorrichtung zum sterilen Befüllen eines Einmalinjektors mit einer definierten Dosis einer Injektionslösung, wobei der Einmalinjektor eine Zylinder-Kolben-Einheit mit einem Zylinder und mit einem beweglichen Kolben umfasst, der mittels einer Stange manuell bedienbar sein kann.

Selberfüll-Einkammersysteme können nur mit Spritzen ohne Kanüle gefüllt werden. Ist jedoch eine Injektionslösung in einer Spritze mit eingeschmolzener Kanüle gelagert, muss der Inhalt erst in eine Spritze ohne Kanüle umgefüllt und gewogen werden, was bei Mustern für die klinische Prüfung wegen der zusätzlichen Fehlermöglichkeiten problematisch ist.

Aus der DE 10 2007 034 871 A1 ist ein Einweginjektor bekannt, bei dem die Injektionslösung aus einer Medikamentenampulle durch einen Schlauchadapter hindurch angesaugt wird. Die Dosierung erfolgt hierbei mittels einer Skala einer Pumpstange, mit der der Kolben einer Zylinder-Kolben-Einheit betätigt wird. Je nach Blickwinkel auf die Skala kann die dosierte Menge variieren.

Der vorliegenden Erfindung liegt daher die Problemstellung zugrunde, ein Verfahren und eine Vorrichtung zum sterilen Befüllen eines Einmalinjektors zu entwickeln, bei dem ohne Zwischenwiegen oder einer Gefahr von Ableseungenauigkeiten der Einmalinjektor mit einer definierten Dosis einer Injektionslösung befüllt werden kann, auch wenn die Dosis sich in einer Spritze mit eingeschmolzener Kanüle befindet.

Diese Problemstellung wird mit den Merkmalen der unabhängigen Ansprüche gelöst. Dazu ist die definierte Dosis der Injektionslösung in einem Behälterinnenraum eines Behälters einer in einem Durchgangsstopfen gehaltenen Nadelspritze bevorratet. Der Zylinderinnenraum ist mit dem Behälterinnenraum durch den Durchgangsstopfen hindurch gas- und flüssigkeitsdicht verbunden.

Die Zylinder-Kolben-Einheit wird durch Zurückziehen ihres Kolbens oder durch Drücken des Nadelspritzenkolbens befüllt.

Weitere Einzelheiten der Erfindung ergeben sich aus den Unteransprüchen und der nachfolgenden Beschreibungen eines schematisch dargestellten Ausführungsbeispiels.
- Figur 1:: Einmalinjektor vor dem Befüllen;
- Figur 2:: Figur 1 mit angesetzter Nadelspritze;
- Figur 3:: Befüllter Einmalinjektor;
- Figur 4:: Durchgangsstopfen;
- Figur 5:: Nadelspritze mit Injektionslösung;
- Figur 6:: Einmalinjektor mit Pumpstange;
- Figur 7:: Einmalinjektor vor dem Auslösen ohne Pumpstange;
- Figur 8:: Einmalinjektor nach dem Auslösen ohne Pumpstange.

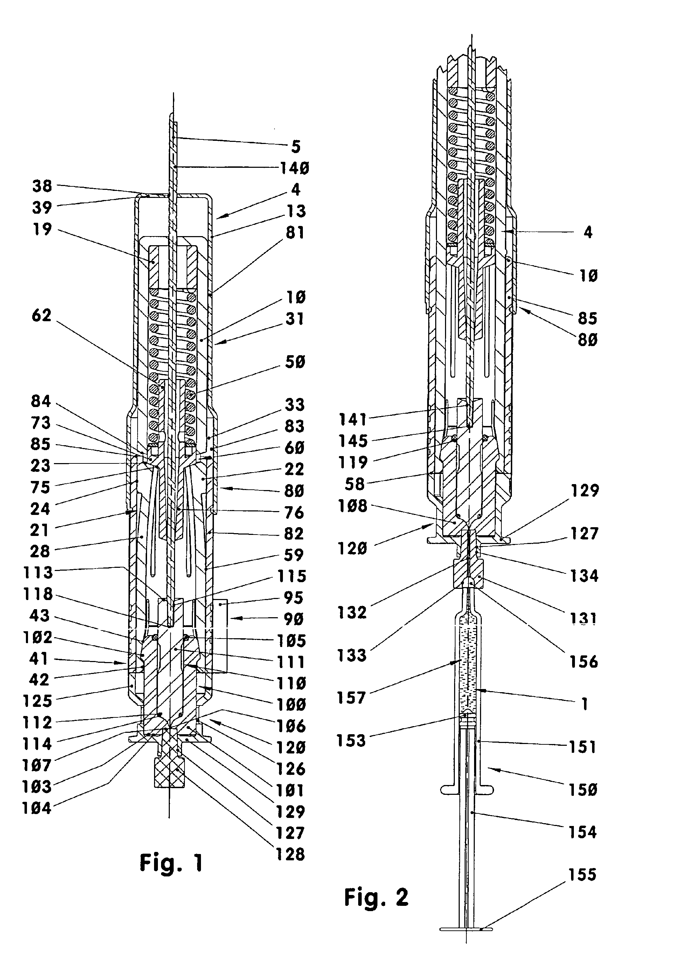

Die Figur 1 zeigt einen Einmalinjektor (4), einen sogenannten Einweginjektor (4). Derartige Injektoren können nur ein einziges Mal eingesetzt werden. Nach dem Gebrauch sind sie gegen einen nochmaligen Gebrauch verriegelt.

Der Einmalinjektor (4) umfasst ein Gehäuse (10), einen Federenergiespeicher (50), einen Kolbenbetätigungsstempel (60) eine Auslöseeinheit (80), eine Zylinder-Kolben-Einheit (100) und einen Originalitätsverschluss (90).

Das Gehäuse (10) ist ein topfförmiger, unten offener Hohlkörper mit obenliegendem Boden (39). Es wird z.B. aus einem glasfaserverstärkten Polyamid durch Spritzgießen gefertigt. Das Gehäuse (10) hat eine weitgehend rohrförmige Gestalt und ist in zwei Funktionsbereiche aufgeteilt, das sind zum einen der obere Mantelbereich (31) und zum anderen der untere Fixierbereich (41). Im Mantelbereich (31) hat das Gehäuse (10) z.B. zwei einander gegenüberliegende fensterartige Durchbrüche (33). Am unteren Rand des einzelnen Durchbruchs (33) ist jeweils ein Druckstab (21) gelenkig gelagert. Der Boden (39) hat eine zentrale Bohrung (38).

Der Druckstab (21) hat beispielsweise auf 80 % seiner Länge die Wandstärke und die Krümmung der Wandung des Gehäuses (10). Dieser Bereich hat unter anderem auch die Funktion eines federelastischen Biegebalkens (28). Er hat einen sichelförmigen Querschnitt.

Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren.

Das hier obere freie Ende des einzelnen Druckstabs (21) wird durch den radial nach außen abstehenden Nocken (22) gebildet. Letzterer hat zumindest eine in Richtung der Mittellinie (5) orientierte Abstützfläche (23) und eine der Mittellinie (5) abgewandte Anlagefläche (24).

Die untere Hälfte des Gehäuses (10) ist von dem hülsenartigen Auslöseelement (82) umgeben. Dieses ist z.B. im Wesentlichen zylindrisch ausgebildet und beispielsweise aus Acrylnitril-Butadien-Styrol-Copolymer (ABS) hergestellt. Das Auslöseelement (82) ist auf der radialen Außenfläche (13) des Gehäuses (10) längs verschiebbar gelagert. Es endet rückwärtig mit einer scharfen Kante (85), die Teil einer stirnseitigen Rücksprungflanke (84) des Auslöseelements (82) ist. Unterhalb der Kante (85) berühren nach Figur 1 die an die Druckstäbe (21) angeformten Nocken (22) mit ihren außen liegenden Anlageflächen (24) sichernd die Innenwandung (59) des Auslöseelements (82).

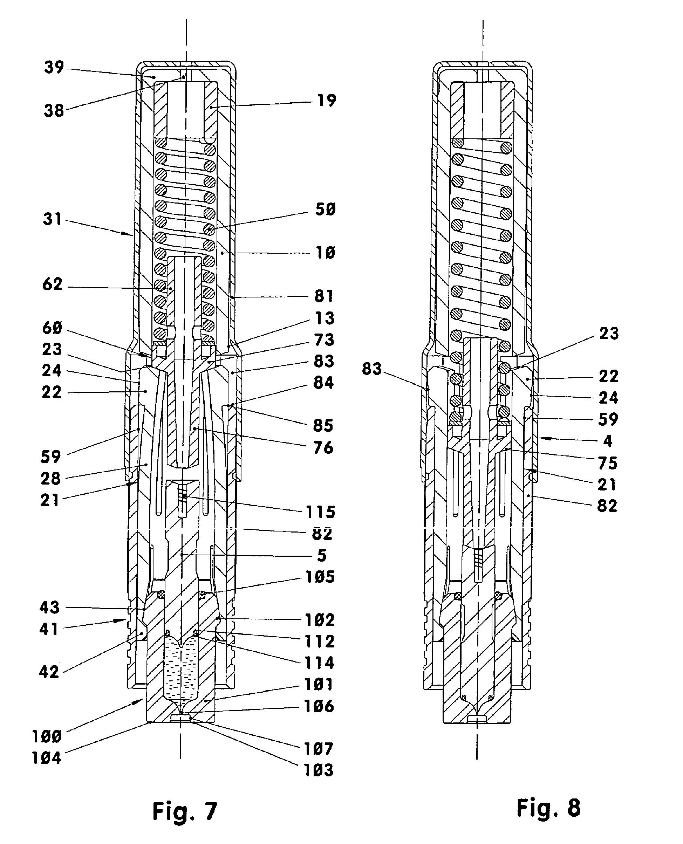

Beispielsweise in der Nähe der Kante (85) ist am Auslöseelement (82) eine Auslösekappe (81) befestigt, die das hintere Ende des Gehäuses (10) vollständig umgibt. Die Auslösekappe (81) umfasst eine umlaufende Aufweitung (83), in der beim Auslösen des Injektors die Nocken (22) aufgenommen werden, vgl. Figur 8. Anstelle dieser Aufweitung (83) können bei einem nichtrotationssymmetrischen Auslöseelement (82) pro Druckstab (21) auch partielle Aufweitungen oder nicht abgedeckte Öffnungen vorhanden sein. Oberhalb der Aufweitung (83) liegt die Auslösekappe (81) gleitfähig an der Außenwandung (13) des Gehäuses (10) an.

Der im Gehäuse (10) angeordnete Kolbenbetätigungsstempel (60) ist in zwei Bereiche aufgeteilt. Der untere Bereich ist der Kolbenschieber (76). Sein Durchmesser ist etwas kleiner als der Innendurchmesser des hinteren Bereichs des Zylinders (101) einer Zylinder-Kolben-Einheit (100). Die untere Stirnfläche des Kolbenschiebers (76) wirkt direkt auf den Kolben (111) dieser Zylinder-Kolben-Einheit (100).

Der obere Bereich des Kolbenbetätigungsstempels (60), der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31). Die untere Stirnseite weist eine um den Kolbenschieber (76) herum angeordnete Bundfläche (75) auf. Sie hat die Form eines Kegelstumpfmantels, dessen Spitzenwinkel ca. 100 bis 140 Grad beträgt. Im dargestellten Ausführungsbeispiel hat die Bundfläche (75) einen Spitzenwinkel von 140 Grad. Die gedachte Spitze des Kegelstumpfmantels liegt auf der Mittellinie (5) im Bereich des Kolbenschiebers (76). Die Bundfläche (75) kann auch sphärisch gekrümmt sein.

Der Kolbenschieber (76) kann selbstverständlich auch als separates, vom Stempelteller (73) getrenntes Bauteil ausgeführt sein. Hierzu ist er dann an der Innenwandung des Gehäuses (10) geführt.

Zwischen dem Stempelteller (73) und dem oben liegenden Boden (39) des Gehäuses (10) sitzt vorgespannt die Schraubendruckfeder (50). Die Schraubendruckfeder (50) stützt sich beispielsweise über eine Distanzhülse (19) am Boden (39) des Gehäuses (10) ab. Die Federkraft der Schraubendruckfeder (50) wird über den Stempelteller (73) auf die Druckstäbe (21) übertragen. Aufgrund der Neigung der Bundfläche (75) werden die Druckstäbe (21) keilgetriebeartig radial nach außen gedrängt. Die Auslösehülse (82) stützt diese Radialkraft dauerhaft ab.

Der Kolbenbetätigungsstempel (60) hat oberhalb des Stempeltellers (73) einen Führungszapfen (62). Letzterer führt die Schraubendruckfeder (50) oder wird durch diese geführt. Unterhalb des Stempeltellers (73) befindet sich zentral in der Verlängerung des Führungszapfens (62) der Kolbenschieber (76).

Unterhalb des Mantelabschnitts (31) befindet sich der Fixierbereich (41) zur Aufnahme der einbaubaren Zylinder-Kolben-Einheit (100). Der Fixierbereich (41) umfasst z.B. acht parallel zur Mittellinie (5) ausgerichtete Federhaken (42). Die Federhaken (42) haben jeweils einen mindestens zweiflankigen Hintergriff (43) zur spielfreien Aufnahme der Zylinder-Kolben-Einheit (100). Die einander gegenüber liegenden Flanken des Hintergriffs (43) schließen einen Winkel von z.B. 90 Winkelgraden ein. Die Länge und die Federrate der Federhaken (42) sind so dimensioniert, dass die Zylinder-Kolben-Einheit (100) ohne plastische Verformung der Federhaken (42) eingebaut werden kann.

Der Zylinder (101), der im Ausführungsbeispiel mit Wasser für Injektionszwecke oder mit einer Injektionslösung (1) befüllbar ist, ist z.B. ein klarsichtiger, dickwandiger Topf, dessen ggf. zylindrische Außenwandung einen beispielsweise umlaufenden Rastring (102) trägt, der an den Flanken des Hintergriffs (43) der Federhaken (42) formsteif anliegt. In der z.B. zylindrischen oder konischen Bohrung des Zylinders (101) sitzt der stangenlose Kolben (111). Der z.B. aus dem Teflon^{®}-Derivat Tetrafluorethylen/Hexafluorpropylen-Copolymer (FEP) hergestellte Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. In der rückseitigen Stirnfläche des Kolbens (111) ist ggf. eine z.B. zylindrische Metallplatte eingelassen. In der Darstellung der Figur 1 sitzt der Kolben (111) in der vorderen Position. Sein oberes Ende ragt aus dem Zylinder (101) heraus. Die Länge des Kolbens (111) ist so gewählt, dass der eingefahrene Kolben (111), vgl. die Figuren 1 und 8, mindestens einen Millimeter über die hintere Oberkante übersteht. Der mittlere Bereich des Kolbens (111) ist tailliert ausgeführt. Der umlaufend taillierte Bereich hat eine Länge, die ca. 30 % der Kolbengesamtlänge entspricht. Der taillierte Bereich hat einen Durchmesser, der 16 bis 20 % kleiner ist als der maximale Zylinderinnendurchmesser im Bereich des lösungsaufnehmenden Zylinderinnenraumes (110). Der vordere Übergang, der zwischen dem taillierten Bereich und dem vorderen, also hier unten liegenden, Kolbenbereich liegt, hat z.B. einen Kegelwinkel von 35 bis 40 Winkelgraden. Der andere, hintere Übergang hat einen Kegelwinkel zwischen 35 und 90 Winkelgraden.

In der rückseitigen, z.B. kegelstumpfmantelförmigen Stirnfläche (113) des Kolbens (111) befindet sich eine zentrische, konische Kolbenausnehmung (115) mit dem Boden (118) zur Ankupplung der Pumpstange (140). Der Kegelwinkel der Kolbenausnehmung (115) beträgt z.B. ein Winkelgrad. Die Pumpstange (140) hat zum Ankuppeln am Kolben (111) an ihrem unteren Ende z.B. ein kegeliges Spitzgewinde (141). Der Kegelwinkel des Spitzgewindes (141) beträgt z.B. sechs Winkelgrade. Der Gewindegang des Spitzgewindes (141) drückt beim Eindrehen der Pumpstange (140) in die Kolbenausnehmung (115) das erforderliche Gegengewinde ein. Der Eindrehvorgang ist beendet, wenn das vordere Ende der Pumpstange (140) mit der schmalen Spitze der kegelstumpfförmigen Stirnseite (145) den Grund (118) kontaktiert.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101). Diese Stirnfläche (103) kann zur Erhöhung der Applikationssicherheit zusätzlich mit einem Klebering (104) versehen werden.

An der unteren Stirnfläche des Auslöseelements (82) liegt die am Zylinder (101) der Zylinder-Kolben-Einheit (100) zentrierte Verschlusskappe (120) an. Die zumindest annähernd zylindrische Außenfläche der Verschlusskappe (120) hat den gleichen Durchmesser wie die ebenfalls zylindrische Außenfläche des Auslöseelements (82) in der Nähe der Stirnseite (58).

Die Verschlusskappe (120) ist ein Becher, der zumindest das untere Viertel der Zylinder-Kolben-Einheit (100) eng anliegend umgibt. Ein Teil der Verschlusskappe (120) liegt mit ihrem Topfbereich (125) an der zylindrischen Außenwandung des Zylinders (101) und an der unteren Stirnfläche (103) mit dem dort befestigten Klebering (104) an.

Der Topfbereich (125) weist zwei einander gegenüberliegende Fenster (126) auf. Die Fenster (126) haben eine Breite, die mindestens dem Durchmesser des Kolbens (111) entspricht. Die Unterkante der Fenster (126) - also die Kanten, die dem tellerartigen Fuß (129) am nächsten liegen - sind in der Höhe des Zylinderbodens (108) angeordnet. Mithilfe der Fenster (126) lässt sich im Durchlicht u.a. die Blasenfreiheit der Zylinderbefüllung prüfen. Am tellerartigen Fuß (129) ist z.B. ein kegelmantelförmiges Rohrstück als Adapteröffnung (127) angeformt. Die Adapteröffnung (127) hat als Innenwandung zumindest bereichsweise einen Luer-Innenkegel. Nach der Figur 1 ist die Adapteröffnung (127) mittels eines Verschlusstopfens (128) steril verschlossen. Der Verschlusstopfen (128) hat hierfür z.B. einen Luer-Außenkegel, mit dem er kraftschlüssig in der Adapteröffnung (127) sitzt und gleichzeitig auch die Ausnehmung (107) des Zylinders (101) steril gas- und flüssigkeitsdicht verschließt.

Zur Sicherung vor dem Auslösen ist die Verschlusskappe (120) über eine Banderole (90) mit dem Auslöseelement (82) des Injektors (4) verbunden. Die Banderole (90) ist ein als Klebeetikett ausgebildeter Originalitätsverschluss.

Die Banderole (90) selbst ist z.B. ein mit einem Klebstoff bereichsweise einseitig beschichteter Papier- und/oder Folienstreifen. Sie besteht beispielsweise aus drei separaten Streifen, die jeweils über eine Perforation oder über eine andere Sollbruchstelle gegeneinander abtrennbar sind.

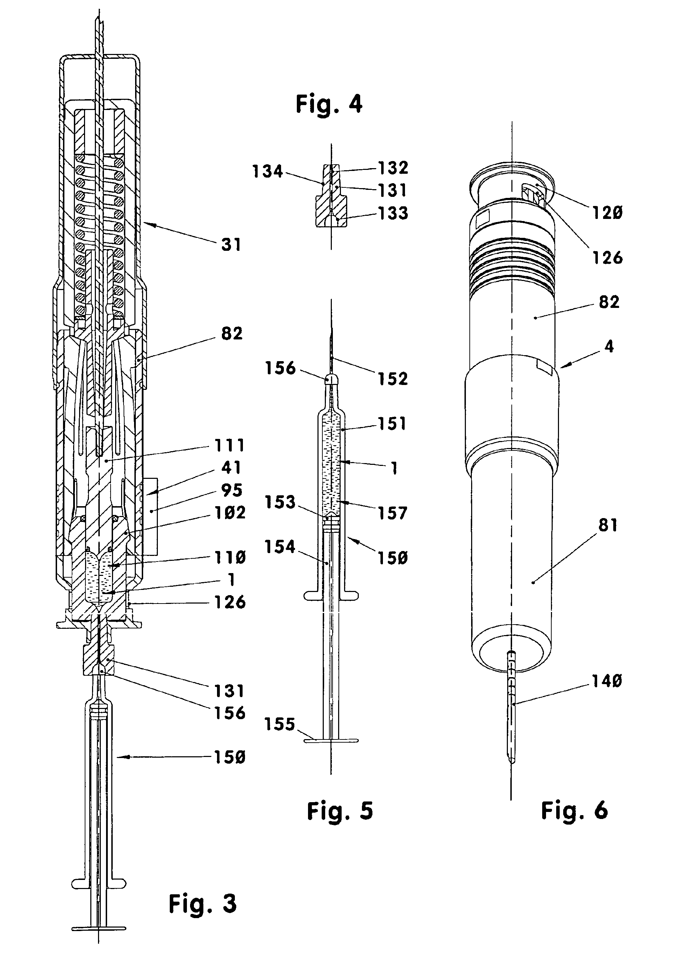

Die Figur 2 zeigt den Einweginjektor (4) mit einer an diesen angesetzten Nadelspritze (150). Die Nadelspritze (150), vgl. Figur 5, hat im Ausführungsbeispiel einen Glaszylinder (151) mit einer eingeschmolzenen Kanüle (152). Die Kanüle (152) kann nur unter Zerstörung des Glaszylinders (151) von diesem getrennt werden. Der in Richtung der Kanüle (152) orientierte Kopf (156) des Glaszylinders (151) ist kalottenförmig ausgebildet. Im Glaszylinder (151) ist ein Kolben (153) mit einer Kolbenstange (154) und einem Betätigungsstempel (155) geführt. Die Nadelspritze (150) ist z.B. mit einer Einzeldosis von beispielsweise 0,4 Millilitern der wirkstoffhaltigen Injektionslösung (1) Clexane befüllt.

Der in der Figur 1 dargestellte verschlusstopfen (128) ist in der Figur 2 durch einen Durchgangsstopfen (131) ersetzt, dessen Bohrung (132) mindestens so lang ist wie die Kanüle (152). Gegebenenfalls kann der Durchgangsstopfen (131) eine Sterilmembran aufweisen und als Originalitätsverschluss der Nadelspritze (150) dienen. Beim Einsetzen in den Injektor (4) zum Befüllen durchstößt die Nadel (152) die Membran. Beispielsweise haftet der Durchgangsstopfen (131) z.B. mit einem Außenkonus (134) kraft- und/oder formschlüssig an der Verschlusskappe (120) und verschließt steril die Ausnehmung (107) des Zylinders (101) gas- und flüssigkeitsdicht gegen die Umgebung. Dieser in der Figur 4 als Einzelteil dargestellte Doppelkonusadapter (131) ist beispielsweise aus einem synthetischen thermoplastischen Kunststoff, z.B. Polyethylen, hergestellt. Er kann jedoch aus nichtrostendem Stahl, Glas, Titan, Naturkautschuk, etc. hergestellt sein. Der Querschnitt der in der Stopfenlängsrichtung orientierten Durchgangsbohrung (132) entspricht beispielsweise mindestens dem Kanülenquerschnitt. An seinem der Düsenbohrung (106) abgewandten Ende hat dieser Luer-Stopfen eine die Durchgangsbohrung (132) umgebende Ausnehmung (133), z.B. einen Innenkonus (133). Diese z.B. konkav ausgebildete Ausnehmung (133) nimmt den Kopf (156) der Nadelspritze (150) gas- und flüssigkeitsdicht auf, während die Durchgangsbohrung (132) die Nadel (152) mit geringem Spiel umgreift. Die Nadelspritze (150) ist im Durchgangstopfen (131) gas- und flüssigkeitsdicht fixiert oder in diesem verrastet.

Um den Einweg-Injektor (4) benutzen zu können, muss die Zylinder-Kolben-Einheit (100) befüllt werden. Hierzu wird die gesamte, in der Nadelspritze (150) gelagerte Injektionslösung (1) durch das Eindrücken des Nadelspritzenkolbens (153) mittels der Kolbenstange (154) oder durch das Zurückziehen des Kolbens (111) mittels der Pumpstange (140) in den Zylinder (101) gedrückt bzw. gesaugt. Die Injektionslösung (1) fließt hierbei aus dem Behälterinnenraum (157) in den mit ihm gegen die Umgebung gas- und flüssigkeitsdicht verbundenen Zylinderinnenraum (110). Beim Füllen durch Saugen wird die Pumpstange (140) manuell entlang ihres gesamten Hubes von der vorderen in die hintere Endlage gezogen. Das hintere Dichtelement (105) verbleibt hierbei ortsfest am Bund (119), auch wenn der taillierte Bereich des Kolbens (111) das Dichtelement (105) passiert. Hat der Kolben (111) seine hintere Stellung erreicht, liegt das Dichtelement (105) wieder steril radial dichtend am Kolben (111) an. Die Kombination aus den Dichtelementen (114) und (105) gewährleistet auch bei der Pumpbewegung des Kolbens (111) einen sterilen Zylinderinnenraum (110). Die Figur 3 zeigt den befüllten Einweginjektor (4). Der Ansaugvorgang wird abgeschlossen, indem die ggf. in den Zylinder (101) angesaugten Gasblasen in bekannter Weise z.B. durch ein geringfügiges Zurückschieben des Kolbens (111) oder des Kolbens (153) - bei nach oben gehaltener Verschlusskappe (120) - entfernt werden. Zur Kontrolle dienen die zwei in der Verschlusskappe (120) angeordneten Fenster (126). Nun kann beispielsweise die z.B. skalierte Pumpstange (140) aus der Ausnehmung (115) des Kolbens (111) herausgedreht und aus dem Gehäuse (10) herausgezogen werden. Im Zylinder (101) der Zylinder-Kolben-Einheit (100) ist nun die definierte Einzeldosis des Arzneimittels (1) gelagert.

Die Nadelspritze (150) mit dem Durchgangsstopfen (131) kann nun von der Verschlusskappe (120) und vom Zylinder (101) abgezogen werden.

Nach dem Beladen und ggf. Entlüften des Zylinders (101) kann zum Zwischenlagern des befüllten Injektors die Düsenbohrung (106) zusammen mit der Ausnehmung (107) mittels eines sterilen Stopfens (128) auch vorn wieder steril gas- und flüssigkeitsdicht verschlossen werden. Auch dieser Stopfen (128) haftet kraft-und/oder formschlüssig in der Verschlusskappe (120).

Zum Entsichern des Einweg-Injektors wird die Abreißbanderole (90) mithilfe der Abreißfahne (95) ringsherum vom Hauptteil getrennt. Die Verschlusskappe (120) wird nun mit dem Stopfen (128) nach unten vom Zylinder (101) abgezogen, vgl. Figur 7.

Nun wird der Injektor auf die Injektionsstelle gesetzt und die hülsenartige Auslöseeinheit (80) nach unten - in Richtung der Injektionsstelle - geschoben. Die Druckstäbe (21) biegen sich elastisch nach außen in ihre eigentliche Ausgangslage. Hierbei rutschen die Nocken (22) über die Kante (85) nach außen in die Aufweitung (83). Die nun nicht mehr verformten Druckstäbe (21) geben den Kolbenbetätigungsstempel (60) frei, so dass sich der Kolben (111) unter der Wirkung des Federelements (50) schlagartig unter Entleeren des Zylinders (101) nach unten bewegt, vgl. Figur 8. Bei der Vorwärtsbewegung des Kolbens (111) vermindert sich die Kolbenreibung zwischenzeitlich, da das rückwärtige Dichtelement (105) beim Passieren des taillierten Kolbenbereiches nicht bremsend anliegt. Die Injektionslösung (1) wird in die Injektionsstelle gefördert.

### Bezugszeichenliste:

- 1: Injektionslösung; Medikament
- 4: Einmalinjektor, Einweginjektor, Injektor
- 5: Mittellinie des Injektors, Längsrichtung

- 10: Gehäuse, einteilig
- 13: Außenfläche, zylindrisch
- 19: Distanzhülse

- 21: Druckstäbe, Stützstäbe; Zughaken
- 22: Nocken
- 23: Abstützfläche
- 24: Anlagefläche
- 28: Biegebalken

- 31: Mantelbereich
- 33: Durchbrüche 38 Bohrung
- 39: Boden

- 41: Fixierbereich für die Zylinder-Kolben-Einheit
- 42: Federhaken 43 Hintergriff

- 50: Federelement, Schraubendruckfeder, Federenergiespeicher
- 58: Stirnseite von (82)
- 59: Innenwandung von (82)

- 60: Kolbenbetätigungsstempel
- 62: Führungszapfen

- 73: Stempelteller
- 75: Bundfläche, konisch
- 76: Kolbenschieber

- 80: Auslöseeinheit
- 81: Auslösekappe
- 82: Auslöseelement
- 83: Aufweitung
- 84: Rücksprungflanke
- 85: Kante, scharfkantig

- 90: Originalitätsverschluss, Banderole, Sicherungselement
- 95: Abreißfahne

- 100: Zylinder-Kolben-Einheit
- 101: Zylinder
- 102: Rastring
- 103: Stirnfläche
- 104: Klebering
- 105: Dichtelement
- 106: Bohrung, Düse
- 107: Ausnehmung in der Stirnfläche
- 108: Zylinderboden

- 110: Zylinderinnenraum
- 111: Kolben
- 112: Ringnut
- 113: Stirnseite, hinten; Konus
- 114: Dichtring, Dichtung, Dichtelement
- 115: Kolbenausnehmung, Bohrung
- 118: Ausnehmungsgrund von (115)
- 119: Bund an (101)

- 120: Verschlusskappe, Klebeversiegelung
- 125: Topfbereich
- 126: Fenster, beidseitig
- 127: Adapteröffnung
- 128: Stopfen
- 129: Fuß

- 131: Doppelkonusadapter, Durchgangsstopfen
- 132: Durchgangsbohrung
- 133: Ausnehmung, Innenkonus, Aufnahmeeinsenkung
- 134: Außenkonus

- 140: Pumpstange
- 141: Kegelgewinde, Spitzgewinde
- 145: Stirnseite, kegelstumpfförmig

- 150: Nadelspritze
- 151: Glaszylinder, Behälter
- 152: Kanüle, Nadel
- 153: Kolben, Nadelspritzenkolben
- 154: Kolbenstange
- 155: Betätigungsstempel
- 156: Kopf
- 157: Behälterinnenraum

## Patentansprüche

1. Verfahren zum sterilen Befüllen eines Einmalinjektors (4) mit einer definierten Dosis einer Injektionslösung (1),
- wobei der Einmalinjektor (4) eine Zylinder-Kolben-Einheit (100) mit einem Zylinder (101) und mit einem Kolben (111) umfasst,
- wobei die definierte Dosis der Injektionslösung (1) zunächst im Behälterinnenraum (157) eines Behälters (151) einer in einem Durchgangsstopfen (131) gehaltenen Nadelspritze (150) bevorratet ist,
- wobei der Zylinderinnenraum (110) mit dem Behälterinnenraum (157) durch den Durchgangsstopfen (131) hindurch gas- und flüssigkeitsdicht verbunden ist und
- wobei die Zylinder-Kolben-Einheit (100) durch Zurückziehen des Kolbens (111) oder durch Drücken des Nadelspritzenkolbens (153) befüllt wird.

2. Vorrichtung zum sterilen Befüllen eines Einmalinjektors (4) mit einer definierten Dosis einer Injektionslösung (1), wobei der Einmalinjektor (4) eine Zylinder-Kolben-Einheit (100) mit einem Zylinder (101) und mit einem beweglichen Kolben (111) umfasst, **dadurch gekennzeichnet,**
- **dass** die definierte Dosis der Injektionslösung (1) in einem Behälterinnenraum (157) eines Behälters (151) einer in einem Durchgangsstopfen (131) gehaltenen Nadelspritze (150) bevorratet ist und
- **dass** der zylinderinnenraum (110) mit dem Behälterinnenraum (157) durch den Durchgangsstopfen (131) hindurch gas- und flüssigkeitsdicht verbunden ist.

3. Vorrichtung nach Anspruch 2, **dadurch gekennzeichnet, dass** am Kolben (111) eine entfernbare Pumpstange (140) befestigt ist.

4. Vorrichtung nach Anspruch 2, **dadurch gekennzeichnet, dass** der Durchgangsstopfen (131) aus einem synthetischen thermoplastischen Kunststoff hergestellt ist.

5. Vorrichtung nach Anspruch 2, **dadurch gekennzeichnet, dass** der Durchgangsstopfen (131) eine Aufnahmeeinsenkung (133) umfasst.

6. Vorrichtung nach Anspruch 2, **dadurch gekennzeichnet, dass** die Nadelspritze (150) einen verschiebbaren Kolben (153) umfasst.
